Europäisches Patentamt

European Patent Office

Office européen des brevets

(19)

(11) **EP 0 939 316 A2**

(12) **DEMANDE DE BREVET EUROPEEN**

(43) Date de publication:
**01.09.1999 Bulletin 1999/35**

(51) Int Cl.[6]: **G01N 33/02**, G01N 21/35

(21) Numéro de dépôt: **99400476.0**

(22) Date de dépôt: **26.02.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **26.02.1998 FR 9802309**

(71) Demandeurs:
- **CENTRE NATIONAL DU MACHINISME AGRICOLE, DU GENIE RURAL, DES EAUX ET DES FORETS (CEMAGREF) F-92160 Antony (FR)**
- **Copa Informatique 13103 Saint Etienne du Gres (FR)**

(72) Inventeurs:
- **Roger, Jean-Michel 34660 Cournonsec (FR)**

- **Bellon-Maurel, Véronique 34790 Grabels (FR)**
- **Fatou, Jean-Michel 34820 Teyran (FR)**
- **Steinmetz, Vincent 08300 Tagnon (FR)**
- **Crochon, Michel 34270 Valflaunes (FR)**
- **Pelouzet, Roger 13103 Saint Etienne du Gres (FR)**
- **Martinez, Antoine 30320 Marguerittes (FR)**

(74) Mandataire: **Joly, Jean-Jacques et al Cabinet Beau de Loménie 158, rue de l'Université 75340 Paris Cédex 07 (FR)**

(54) **Procédé et installation pour la mesure de la teneur, notamment en sucre, de fruits et légumes**

(57) L'intérieur des fruits ou légumes est illuminé à travers leur surface par un rayonnement lumineux infrarouge. Les données spectrales représentatives du rayonnement rétrodiffusé sont recueillies et utilisées pour fournir une valeur de la teneur recherchée par rapprochement avec au moins un modèle de prédiction préétabli. Une pluralité de mesures sont effectuées par illumination des fruits ou légumes à caractériser au moyen d'un faisceau de fibres optiques dont une extrémité est appliquée sur la surface desdits fruits ou légumes, et la valeur de la teneur recherchée est élaborée à partir de la moyenne des mesures effectuées et en s'affranchissant de l'influence de la température réelle des fruits ou légumes à caractériser.

FIG.1

EP 0 939 316 A2

## Description

<u>Domaine de l'invention</u>

L'invention concerne la mesure de la teneur de fruits ou légumes en une matière déterminée, en particulier la teneur en sucre. L'invention convient également à la mesure de teneurs en autres matières, par exemple la teneur en matières grasses.

Les domaines d'application de l'invention sont notamment le tri automatique des fruits, en vue de leur conditionnement, la réception ou l'agréage de lots de fruits et la sélection de fruits pour la cueillette.

<u>Arrière-plan de l'invention</u>

Il existe une demande pour que le tri de fruits ou légumes en vue de leur conditionnement et de leur expédition soit effectué non seulement en fonction de caractéristiques visibles, notamment le calibre et la couleur, mais aussi de caractéristiques moins visibles, telles la valeur gustative et la maturité.

Pour beaucoup de fruits et légumes, la mesure de la teneur en sucre fournit une information utile pour l'appréciation de la valeur gustative ou du degré de maturité. Dans certains cas, la mesure de la teneur en une autre matière peut être plus représentative, par exemple la teneur en matières grasses pour les avocats.

Une technique bien connue consiste à effectuer une mesure par réfractométrie sur du jus ou de la pulpe prélevé sur le fruit ou légume. Toutefois, à l'exception de fruits ou légumes à peau épaisse, tels que les cucurbitacées, par exemple les melons, cette mesure invasive est destructrice car elle annule la valeur marchande du produit. En outre cette technique est incompatible avec des installations de tri automatique fonctionnant à cadence élevée, ou avec des systèmes de sélection de fruits ou légumes lors de la cueillette, en particulier lorsque celle-ci est mécanisée.

Des techniques non invasives de mesure de taux de sucre dans des fruits ou légumes ont déjà été décrites.

Ainsi, il a été proposé par la société japonaise Mitsui Mining & Smelting Co. Ltd. d'éclairer un fruit ou légume par un rayonnement lumineux dans le domaine du visible ou du proche infrarouge et d'évaluer le taux de sucre par mesure de la réflectance ou de la transmittance dans diverses longueurs d'onde. Il peut être fait référence notamment aux documents JP 1-028 544, JP 1-301 147, JP 4-104 041, JP 6-300 680 et JP 6-300 689.

Avec ces techniques, il est nécessaire d'utiliser des sources de rayonnement puissantes. Un autre inconvénient réside dans la sensibilité à l'environnement (lumières parasites). De plus, la mesure de transmittance n'est envisageable qu'avec quelques types de fruits ou légumes seulement.

Un procédé de mesure de taux de sucre dans des fruits par spectrométrie infrarouge est décrit par ailleurs dans un article de Véronique Bellon et al. publié dans "Applied Spectrometry", volume 47, n° 7, 1993.

Un faisceau de fibres optiques en forme d'Y permet d'illuminer une zone limitée de la chair au travers de la surface d'un fruit par un rayonnement dans le proche infrarouge et de recueillir le rayonnement rétrodiffusé. Les caractéristiques d'absorption du rayonnement infrarouge varient selon le taux de sucre dans la partie du fruit située sous la zone de surface illuminée. L'analyse du spectre du rayonnement rétrodiffusé permet donc une évaluation du taux de sucre par rapprochement avec un modèle de prédiction préalablement établi en rapprochant des données spectrales de fruits de référence avec leur teneur en sucre mesurée par ailleurs.

Un tel procédé a pour avantages d'être moins sensible aux lumières parasites environnantes et de ne pas requérir de source d'éclairement très puissante.

Toutefois, il a été observé par le demandeur que ce procédé, expérimenté en laboratoire, ne permet pas toujours d'obtenir des résultats satisfaisants lorsqu'il est mis en oeuvre industriellement.

Il est connu aussi du document EP 0 317 121 d'effectuer une mesure de teneur en sucre par illumination d'une zone de surface d'un fruit successivement avec deux niveaux d'intensité différents dans le proche infrarouge, et calcul de la différence entre les deux spectres d'absorption recueillis. Ce procédé permet de déterminer le taux de sucre à une certaine profondeur, en éliminant l'influence de la couche épidermique du fruit.

Ce procédé, de même que les précédents, ne permet pas toutefois de s'affranchir d'imprévisions qui peuvent se produire lorsque la mesure est effectuée dans une zone de surface particulière non représentative de l'ensemble du fruit ou légume, par exemple au niveau d'un pédoncule, d'un calice ou d'une tache sur la peau, ou lorsque la teneur à mesurer varie sensiblement au sein d'un même fruit ou légume.

<u>Objets et résumé de l'invention</u>

L'invention a pour but de fournir un procédé industriel de mesure de la teneur de fruits ou légumes en une matière déterminée, notamment en sucre, par spectrométrie infrarouge.

L'invention a plus particulièrement pour but de fournir un procédé ayant une robustesse accrue, c'est-à-dire capable de fournir des mesures de teneur avec une précision satisfaisante, et ce de façon fiable et répétitive, lorsqu'il est utilisé dans des installations industrielles de tri, de conditionnement ou de cueillette automatisées ou semi-automatisées.

L'invention a aussi pour but de fournir une installation pour la mise en oeuvre du procédé.

Selon l'invention, un procédé pour la mesure de la teneur de fruits ou légumes en une matière déterminée comprend l'illumination de l'intérieur des fruits ou légumes au travers de leur surface par un rayonnement lumineux infrarouge, le recueil de données spectrales représentatives du rayonnement rétrodiffusé, et le traitement des données spectrales recueillies pour fournir une valeur de la teneur recherchée par rapprochement avec au moins un modèle de prédiction préétabli, et est caractérisé en ce qu'une pluralité de mesures sont effectuées par illumination des fruits ou légumes à caractériser au moyen d'un faisceau de fibres optiques dont une extrémité est appliquée sur la surface desdits fruits ou légumes, et la valeur de la teneur recherchée est élaborée à partir de la moyenne des mesures effectuées et en s'affranchissant de l'influence de la température réelle desdits fruits ou légumes.

Le procédé selon l'invention peut être mis en oeuvre sur des fruits ou légumes pris individuellement ou sur des fruits ou légumes pris en lot.

Le fait d'effectuer plusieurs mesures dans des zones différentes d'un fruit ou légume ou sur des fruits ou légumes différents d'un même lot permet de fournir un résultat s'approchant d'une valeur moyenne de la teneur recherchée dans tout le fruit ou légume ou dans tout le lot, notamment lorsque la probabilité de mesure extrême est rendue plus élevée par la présence de taches ou une variation importante de couleur sur la surface, ou par une variation importante, et courante, du degré de maturité au sein d'un même fruit ou légume, ou d'un même lot.

De préférence, dans le cas de la caractérisation individuelle d'un fruit ou légume au moins deux mesures sont effectuées dans des zones sensiblement éloignées du fruit ou légume. Plusieurs mesures peuvent être effectuées simultanément ou séquentiellement, par exemple, dans ce dernier cas, en modifiant la position du fruit ou légume à caractériser.

Une autre caractéristique avantageuse du procédé selon l'invention tient à ce que l'illumination est réalisée au moyen d'un faisceau de fibres optiques dont l'extrémité est appliquée sur la surface du fruit ou légume. L'influence de lumières parasites environnantes peut ainsi être évitée et le caractère reproductible des mesures effectuées est amélioré.

De préférence, cette application de l'extrémité du faisceau de fibres est réalisée au moyen d'une force de rappel élastique s'exerçant sur l'extrémité du faisceau de fibres en direction de la surface du fruit ou légume.

De préférence encore, l'extrémité du faisceau de fibres est appliquée en direction sensiblement normale à la surface du fruit ou légume.

Une autre caractéristique avantageuse du procédé selon l'invention tient à ce que la valeur de la teneur recherchée est en outre déterminée en s'affranchissant de l'influence de la température du fruit ou légume, ou du lot de fruits ou légumes, à caractériser.

En effet, la mesure peut être gravement entachée d'erreur en cas d'écart important entre la température effective des fruits ou légumes à caractériser pour lesquels des données spectrales sont recueillies et la température à laquelle a été établi le modèle de prédiction rapproché de ces données spectrales.

Selon un premier mode de mise en oeuvre du procédé, on sélectionne un modèle de prédiction en fonction de la température, effective des fruits ou légumes parmi un ensemble de modèles de prédiction préétablis pour lesdits fruits ou légumes, pour différentes températures.

Selon un deuxième mode de mise en oeuvre du procédé, on élabore une première valeur de la teneur par rapprochement avec un modèle de prédiction préétabli pour une température donnée, et on détermine la valeur de la teneur recherchée à partir de la première valeur élaborée selon une relation prédéterminée fonction de la température des fruits ou légumes.

Selon un troisième mode de mise en oeuvre du procédé, on effectue un rapprochement entre les données spectrales recueillies et un ensemble de modèles de prédiction préétablis pour lesdits fruits ou légumes, pour différentes températures, le taux de corrélation le plus élevé étant alors obtenu avec le modèle de prédiction correspondant à la température la plus proche de la température réelle des fruits ou légumes.

L'invention a aussi pour but de fournir une installation permettant la mise en oeuvre du procédé.

Selon l'invention, une telle installation comprenant une source de rayonnement infrarouge, un dispositif de spectrométrie infrarouge, des moyens optiques reliés à la source et au dispositif de spectrométrie pour illuminer une surface par le rayonnement de la source et acheminer un rayonnement rétrodiffusé au dispositif de spectrométrie, et une unité de traitement reliée au dispositif de spectrométrie pour recueillir un ensemble de données spectrales représentatives d'un rayonnement rétrodiffusé et fournir une valeur de la teneur recherchée par rapprochement avec au moins un modèle de prédiction préétabli, est caractérisée en ce que l'unité de traitement comprend des moyens pour fournir la valeur de teneur recherchée par moyenne de mesures à partir d'une pluralité d'ensembles de données spectrales recueillis, et en s'affranchissant de l'influence de la température réelle de fruits ou légumes à caractériser, et les moyens optiques comprennent un faisceau de fibres optiques dont une extrémité libre est associée à des moyens de rappel élastiques pour solliciter élastiquement l'extrémité du faisceau de fibres vers une surface d'un fruit ou légume.

Avantageusement, l'extrémité libre du faisceau de fibres optiques peut être montée librement pivotante sur un support et une pièce de guidage est montée sur la partie de faisceau commun pour permettre, par contact avec une surface, une orientation automatique de l'axe de la partie d'extrémité libre du faisceau de fibres optiques dans une direction sensiblement normale à la surface du fruit ou légume, par pivotement par rapport au support.

D'autres particularités et avantages du procédé et d'une installation permettant sa mise en oeuvre ressortiront à la lecture de la description faite ci-après, à titre indicatif mais non limitatif.

Brève description des dessins

Il sera fait référence aux dessins annexés sur lesquels:

- la figure 1 illustre très schématiquement un mode de réalisation d'une installation permettant la mise en oeuvre du procédé selon l'invention;
- la figure 2 est une vue de détail en perspective et à échelle agrandie du montage de l'extrémité du faisceau de fibres optiques amené au contact de la surface d'un fruit dans l'installation de la figure 1;
- la figure 3 est une vue en coupe du montage de la figure 2;
- les figures 4 à 8 et 10 sont des organigrammes relatifs à différents enchaînements d'opérations réalisées par l'unité de traitement de l'installation de la figure 1 ;
- la figure 9 montre la variation du spectre d'absorbance d'un fruit en fonction de la température ;
- la figure 10 est une vue d'une variante de réalisation des moyens d'illumination de la surface d'un fruit et de recueil du rayonnement rétrodiffusé; et
- la figure 11 est un organigramme relatif à une variante du processus de rapprochement entre des données spectrales recueillies et des modèles de prédiction.

Description détaillée d'un mode de réalisation

La description qui suit fait référence, par souci de simplicité, à la mesure de la teneur en sucre de fruits. Comme déjà indiqué, et comme cela apparaîtra immédiatement à l'homme de métier, l'invention est tout aussi applicable à la mesure de la teneur en sucre de légumes, de même qu'à la mesure de la teneur de fruits ou légumes en d'autres matières, notamment en matières grasses.

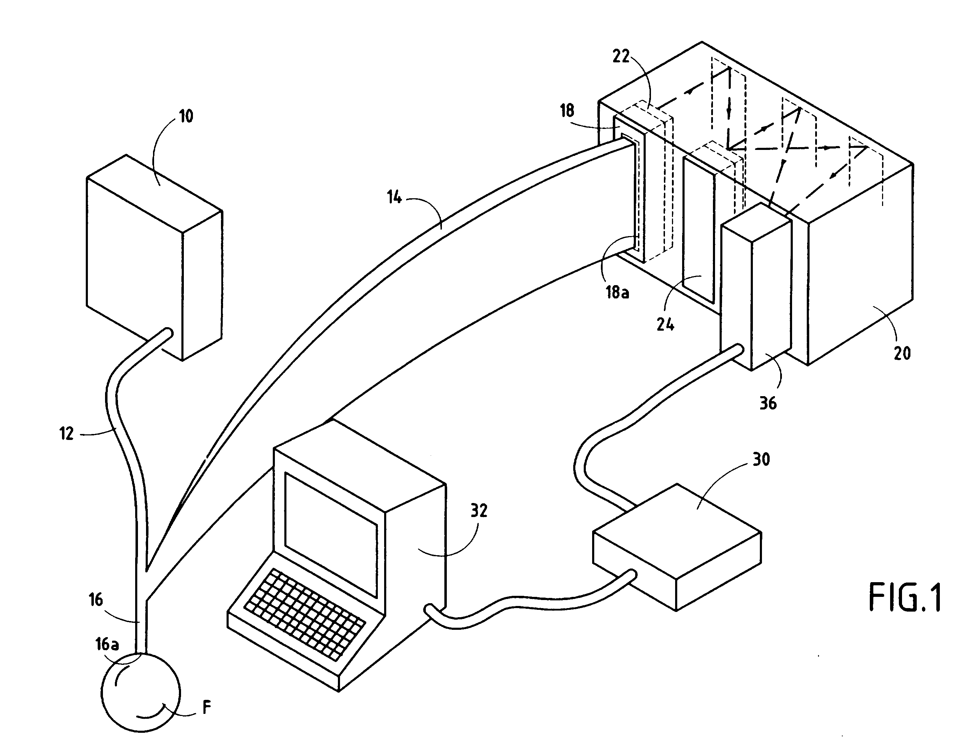

Dans l'installation de la figure 1, une source 10 produisant un rayonnement lumineux dans le domaine des infrarouges est couplée à une branche 12 d'un faisceau de fibres optiques en forme de Y. La branche 12 achemine le rayonnement de la source jusqu'à la surface d'un fruit F, tandis que l'autre branche 14 du faisceau de fibres optiques recueille le rayonnement rétrodiffusé par le fruit pour l'acheminer vers un spectromètre 20. Les branches 12, 14 sont réunies sur une partie terminale de leur trajet pour former un tronçon commun 16 dont l'extrémité 16a est dirigée vers la surface du fruit. Dans le tronçon 16, les fibres émettrices et collectrices sont mélangées.

A l'extrémité de la branche 14 reliée au spectromètre, les fibres collectrices sont disposées en ligne, en regard d'une fente 18a d'un connecteur 18. Un filtre 22 est disposé en entrée du spectromètre afin de couper les longueurs d'onde du domaine visible, par exemple inférieures à 600 nm.

Le rayonnement lumineux traversant la fente 18a et le filtre 22 est envoyé sur un réseau de diffraction 24 à champ plan. L'image diffractée est réfléchie vers un réseau 26 de photodétecteurs situé dans le plan focal, par exemple une caméra à dispositifs à couplage de charge (CCD).

L'image enregistrée par la caméra 26 représente le spectre de rayonnement rétrodiffusé par le fruit F, après filtrage. Cette image, mémorisée, est reçue par une unité de traitement 30 reliée à la caméra 26. Un ensemble écran-clavier 32 assure l'interface entre l'unité de traitement 30 et un opérateur.

Une installation du type de celle brièvement décrite ci-dessus est connue en elle-même, notamment de l'article précité de Véronique Bellon et al.

On notera, en variante, que le rayonnement rétrodiffusé pourra être recueilli par une sphère d'intégration ayant une ouverture destinée à être amenée au contact du fruit pour laisser passer le rayonnement incident amené par un premier faisceau de fibres optiques, et ayant une sortie distincte raccordée à un deuxième faisceau de fibres optiques pour recueillir le rayonnement rétrodiffusé intégré par la sphère. L'utilisation de sphères d'intégration est connue en elle-même. On pourra par exemple se référer au document EP 0 317 121 déjà cité.

L'analyse par spectrométrie infrarouge est effectuée dans un domaine couvrant le proche infrarouge (800 nm-1100 nm) et s'élargissant de préférence vers l'infrarouge plus lointain (800 nm-2500 nm) du fait de la disponibilité de photodétecteurs fonctionnant à plus de 1100 nm.

La source de rayonnement utilisée 10 est par exemple une lampe halogène tungstène émettant dans le domaine 400 nm-2500 nm.

Le spectromètre infrarouge 20, avec le réseau 26 de photodétecteurs, est un produit disponible dans le commerce. On utilisera par exemple l'appareil proposé par la Société ORIEL sous la référence "MULTISPEC".

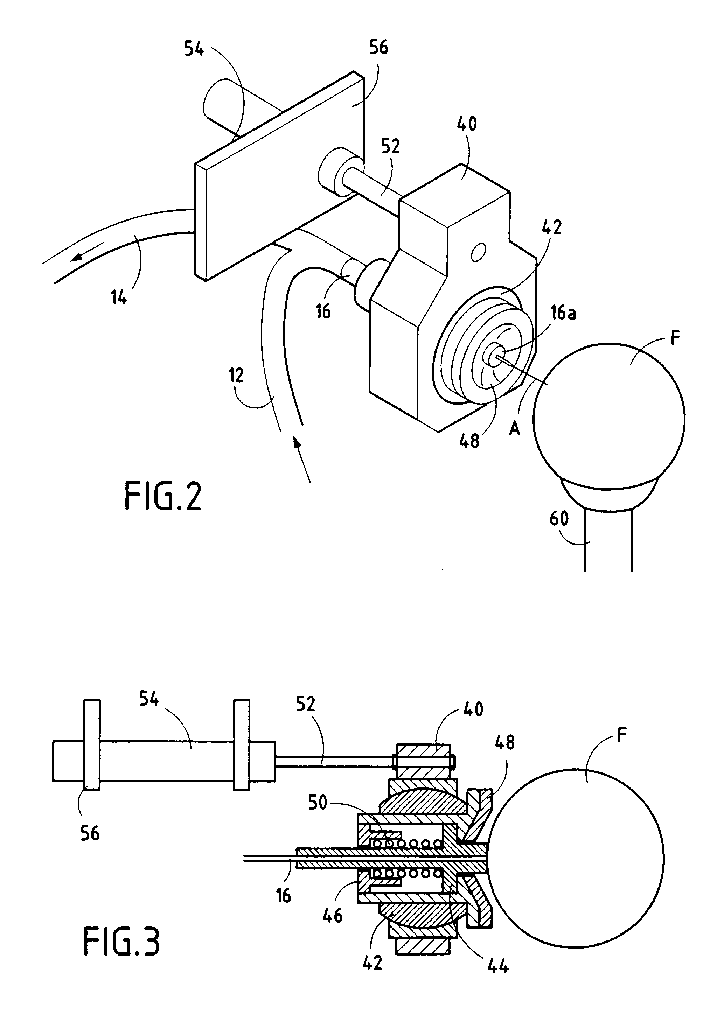

Afin de limiter l'influence des lumières parasites environnantes et la puissance du rayonnement délivré par la source 10, il est nécessaire d'amener l'extrémité 16a du faisceau de fibres optiques au contact de la surface du fruit. En tout état de cause, la distance entre l'extrémité 16a et la surface du fruit doit être, pour un cycle de mesures, constante et égale à celle existant lors de l'établissement du modèle de prédiction. Il est préférable en outre que le rayonnement soit issu de l'extrémité 16a du faisceau de fibres dans une direction sensiblement normale à la surface du fruit. A cet effet, on peut utiliser le montage illustré par les figures 2 et 3 (non montré sur la figure 1).

La partie d'extrémité du tronçon de faisceau 16 est montée dans une tête de mesure 40 par l'intermédiaire d'une articulation à rotule 42 permettant un pivotement libre du tronçon 16 dans la tête 40 dans une plage angulaire. Un manchon 44 est serti sur le tronçon de faisceau 16 au voisinage de l'extrémité 16a. Le manchon 44 est relié, à son extrémité arrière, à une bague 46, montée coulissante sur le tronçon de faisceau 16, avec interposition d'un ressort 50, de sorte qu'un mouvement relatif est autorisé entre l'extrémité 16a et la tête de mesure 40 parallèlement à l'axe A de la partie d'extrémité du tronçon de faisceau 16. Une pièce de guidage 48, telle qu'un cône de centrage entourant l'extrémité 16a, est fixée sur la bague 46. La tête 40 est fixée à l'extrémité de la tige 52 d'un vérin 54 dont le cylindre est monté sur un support 56.

Un fruit F étant maintenu en position par un organe de support 60, le vérin 54 est actionné pour rapprocher la tête de mesure de la surface du fruit. Après venue de l'extrémité 16a au contact de la surface du fruit (figure 3), et compression du ressort 50, la venue du cône de centrage 44 au contact de la surface du fruit permet, par rotation de la rotule 42, une orientation automatique de l'axe A dans une direction sensiblement normale à la surface du fruit. On notera que le ressort 50 permet d'amortir le choc du contact avec le fruit pour ne pas abîmer celui-ci.

Bien que l'on ait envisagé ci-avant l'utilisation d'une rotule, d'autres types d'articulations pourront être utilisés, par exemple une simple articulation élastique.

Selon une variante de réalisation, une mesure peut être effectuée non pas en amenant le fruit vers la tête de mesure, mais en amenant la tête de mesure vers le fruit, alors que celui-ci repose par exemple sur un tapis de convoyeur. Il est alors possible d'effectuer la mesure alors que le fruit est en mouvement, en déplaçant la tête de mesure en synchronisme avec le convoyeur.

Dans d'autres cas, l'amenée du fruit en position de mesure peut être réalisée manuellement. Le fruit est alors appliqué à la main contre la tête de mesure, et il n'est pas nécessaire de prévoir le vérin 54.

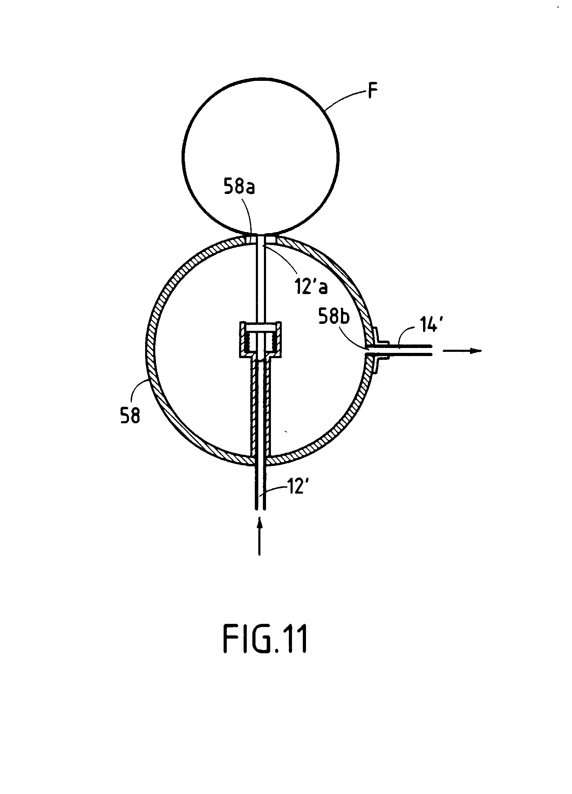

On notera que l'amenée d'un faisceau de fibres optiques au contact de la surface du fruit, avec application de moyens de rappel élastique, et de préférence en direction normale par rapport à la surface du fruit, est aussi réalisée lorsque le rayonnement rétrodiffusé est recueilli par une sphère d'intégration. Dans ce cas, le faisceau de fibres optiques amenant le rayonnement incident traverse la sphère d'intégration jusqu'au contact avec la surface du fruit. Un tel mode de réalisation est montré par la figure 11. Un faisceau de fibres optiques 12' amenant le rayonnement incident traverse une sphère d'intégration 58 jusqu'à une ouverture 58a de celle-ci sur laquelle est amené un fruit F. L'extrémité 12'a du faisceau 12' est sollicitée élastiquement en direction de la surface du fruit F de manière à être au contact de celui-ci, avec une direction sensiblement normale à la surface du fruit. Le rayonnement rétrodiffusé recueilli à travers l'ouverture 58a est intégré dans la sphère 58 d'où il est prélevé par un faisceau de fibres 14' se raccordant à une ouverture 58b de la sphère.

Le fonctionnement de l'unité de traitement 30 recevant des données spectrales représentatives du rayonnement rétrodiffusé par le fruit sera maintenant décrit.

Une caractéristique de l'invention tient à ce que l'évaluation du taux de sucre est effectuée par combinaison de plusieurs mesures, une mesure ne fournissant qu'une indication valable pour une partie limitée du fruit située sous la surface de la peau dans la zone illuminée.

L'évaluation du taux de sucre est aussi effectuée en s'affranchissant de l'influence de la température du fruit. En effet, pour un même taux de sucre, la caractéristique d'absorption du rayonnement infrarouge peut varier de façon très sensible avec la température. Or, dans le cas par exemple de fruits devant être triés, la température peut varier de façon importante entre une valeur basse, lorsqu'ils viennent d'être extraits d'une chambre froide et une valeur haute, lorsqu'ils sont à température ambiante. En préalable à un cycle de mesure, une information représentative de la température des fruits à caractériser peut être mémorisée dans l'unité de traitement. Cette information de température est par exemple saisie par l'opérateur. Il est également possible de mesurer en permanence la température des fruits. Il est encore possible, sans qu'il soit nécessaire de connaître la température des fruits, d'effectuer un rapprochement des données spectrales recueillies avec un ensemble de modèles de prédiction préétablis à différentes températures.

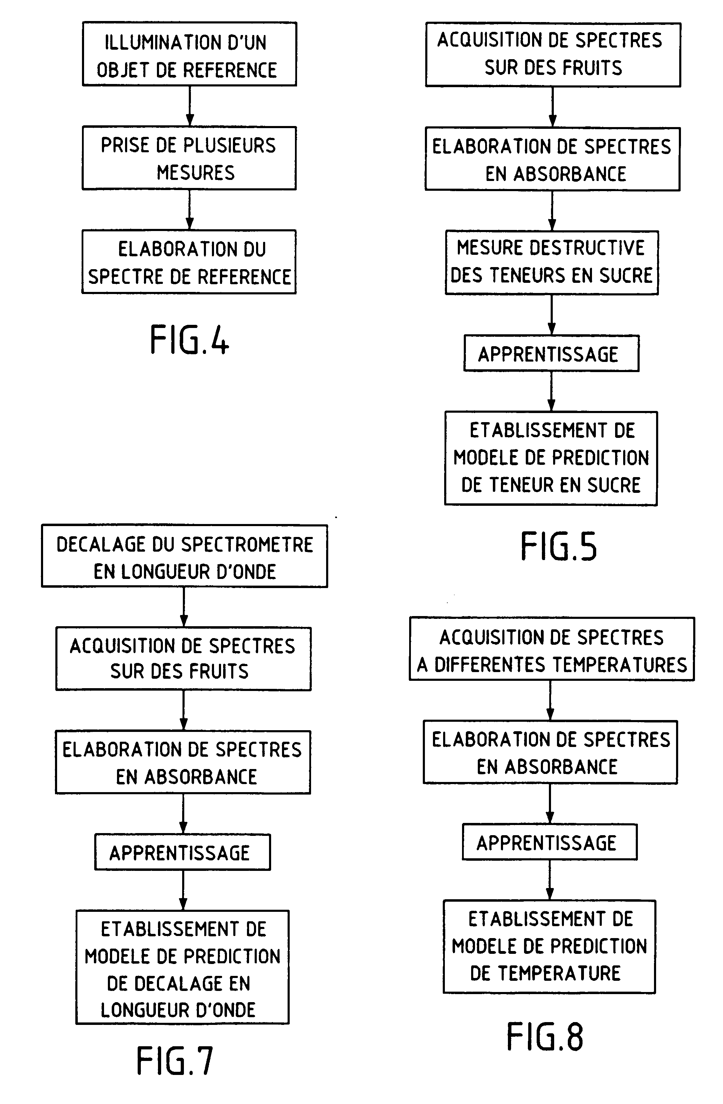

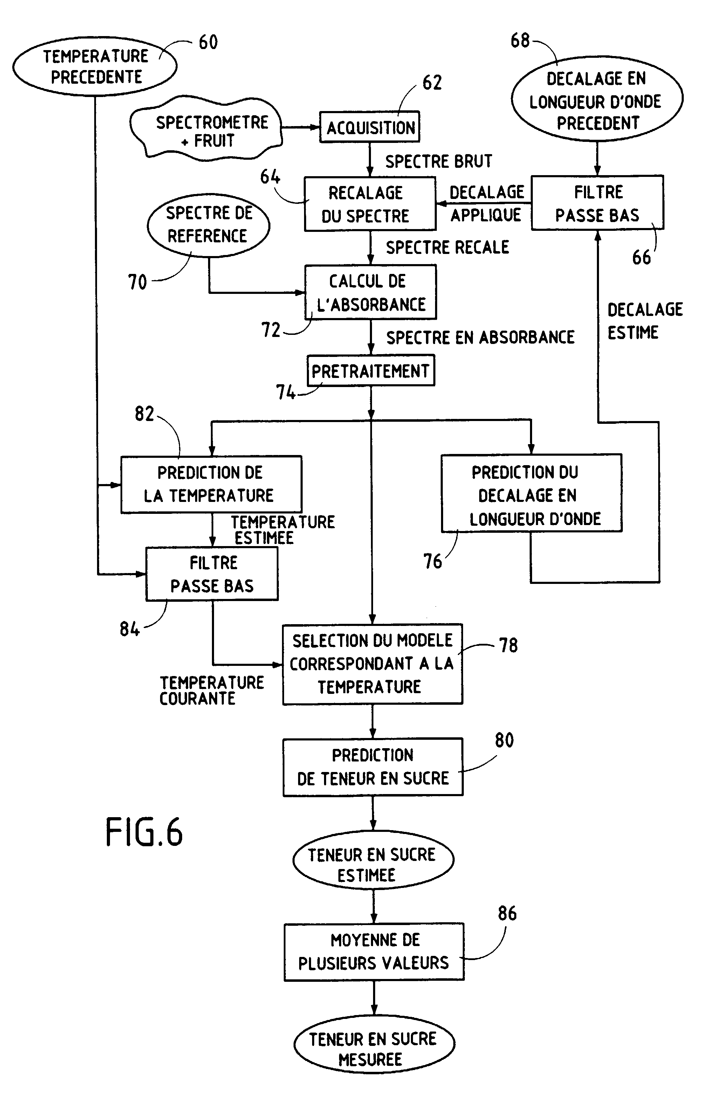

Etablissement d'un spectre de référence (figure 4)

En préalable aux opérations de calibration (établissement de modèles de prédiction) et de mesure effective, un spectre de référence est enregistré dans une situation où le rayonnement émis par la source est réfléchi pratiquement totalement (pas d'absorption du rayonnement).

A cet effet, en place d'un fruit ou légume, et dans la même position que celui-ci, on illumine un objet de référence ayant une surface à haut pouvoir diffusant, réfléchissant le rayonnement incident, sans absorption, et par exemple une forme géométriquement proche de celle des fruits à caractériser. Ainsi, pour des pommes ou pêches, on peut utiliser une boule de couleur blanche telle qu'une boule de billard.

Afin de s'affranchir d'éventuelles singularités superficielles de l'objet de référence, plusieurs prises de mesures sont de préférence effectuées en divers endroits de sa surface et les résultats sont combinés pour élaborer le spectre de référence.

Celui-ci permet, pour chaque spectre recueilli représentatif du rayonnement rétrodiffusé par un fruit, de déterminer le spectre en absorbance par division, longueur d'onde par longueur d'onde, du spectre recueilli par le spectre de référence, méthode connue par ailleurs.

## Calibration (figure 5)

L'évaluation de la teneur en sucre étant effectuée par rapprochement entre des données spectrales recueillies représentatives du rayonnement rétrodiffusé et au moins un modèle de prédiction, une opération préalable d'établissement d'un ou plusieurs modèles de prédiction est nécessaire.

A cet effet, pour un type de fruit donné, un jeu de données est constitué, contenant un lot de spectres dont chacun correspond à une teneur en sucre.

A cet effet, on utilise un ensemble de fruits permettant de recouvrir une plage de teneur en sucre représentative de ce type de fruits (par exemple, de 7 à 20 degrés Brix pour des pommes).

A chaque fois qu'un spectre est recueilli représentant le rayonnement rétrodiffusé par une partie d'un fruit, une mesure destructrice de teneur en sucre est réalisée par prélèvement dans cette partie du fruit.

Les spectres de référence recueillis sont prétraités par division par le spectre de référence, longueur d'onde par longueur d'onde, et les spectres en absorbance obtenus sont mémorisés en relation avec les valeurs correspondantes des teneurs en sucre mesurées.

Une méthode d'apprentissage est mise en oeuvre au moyen de l'unité de traitement 30, qui utilise ce jeu de données pour trouver une relation entre les spectres et les teneurs en sucre correspondantes.

On utilise par exemple la méthode classique des Moindres Carrés Partiels (plus connue sous l'appellation PLS, ou "Partial Least Squares").

Le résultat de cet apprentissage est un modèle de prédiction constitué de:

- un ensemble W de n spectres,
- un ensemble P de n spectres, et
- un ensemble Q de n nombres,

où n est le nombre de facteurs du modèle, n étant choisi pour assurer une précision optimale. En pratique, n est compris entre 5 et 8.

La division des spectres recueillis par le spectre de référence permet d'améliorer l'efficacité de la méthode d'apprentissage, et de s'affranchir de variations de l'intensité lumineuse de la source.

On notera que le processus de calibration décrit ci-avant est d'un type bien connu en lui-même.

La calibration pourra être répétée pour différentes températures de fruit et éventuellement pour différentes natures de fruits.

## Prédiction de la teneur en sucre

La prédiction de la teneur en sucre, lors d'une mesure ultérieure se fait par rapprochement entre le spectre en absorbance S recueilli à partir du rayonnement rétrodiffusé et le modèle de prédiction. Ce rapprochement est effectué de façon connue par n itérations.

A chaque itération, les calculs suivants sont faits:

$$x = S.W_i$$

$$S = S - x * P_i$$

$$C = C + x * Q_i,$$

i étant un entier variant de 1 à n, . étant le produit scalaire, * étant le signe de multiplication, et C étant la teneur en sucre estimée.

Une autre possibilité d'effectuer le rapprochement consiste à réaliser celui-ci en se limitant à un ensemble limité de longueurs d'onde du spectre en absorbance et du modèle de prédiction. Cet ensemble est prédéterminé par mise en oeuvre d'un algorithme génétique, dont le principe est bien connu en soi. A cet effet, on réalise plusieurs prédictions en utilisant à chaque fois un groupe formé d'un nombre limité de longueurs d'onde choisies de façon aléatoire. Ces groupes constituent des gênes initiaux, dont le nombre est au moins égal à 2. Pour chaque prédiction, on relève l'erreur entre la valeur obtenue et celle mesurée de façon invasive. On effectue ensuite des croisements des gênes initiaux jusqu'à extraire un ensemble de longueurs d'onde donnant l'erreur la mois élevée, ensemble qui sera ensuite utilisé lors des mesures effectives.

## Evaluation de la teneur en sucre (figures 6 à 9)

En préalable à une série de mesures, la température des fruits à caractériser, ainsi que, le cas échéant, la nature de ceux-ci, sont saisies par un opérateur au moyen du terminal 32, ou envoyées d'un ordinateur central ou d'un autre système d'acquisition de données. La température est enregistrée en mémoire 60 (figure 6). La saisie de la nature des fruits commande la sélection de modèles de prédiction utilisés dans le processus, l'ordinogramme de la figure 6 étant représenté pour une nature de fruit donnée.

Les données spectrales représentatives du rayonnement rétrodiffusé par un fruit et reçues du spectromètre sont acquises (étape 62), fournissant un spectre brut sous forme numérique.

Une étape 64 de recalage du spectre est ensuite éventuellement réalisée pour tenir compte d'un possible décalage en longueur d'onde du spectromètre. Un tel décalage peut résulter par exemple de vibrations appliquées au spectromètre.

La valeur de décalage appliquée sur le spectre brut est calculée par rapprochement avec un modèle de prédiction. Ce modèle est élaboré (figure 7) de la même façon que le modèle de prédiction de teneur en sucre. Un jeu de données est constitué contenant un lot de spectres acquis sur différents fruits, présentant des teneurs en sucre variées, en décalant artificiellement le spectromètre par incréments de longueurs d'onde dans une plage représentative des décalages à détecter, par exemple dans une plage de -3 nm à +3 nm à partir d'un décalage nul. Les spectres recueillis sont traités par division par le spectre de référence afin de fournir des spectres en absorbance qui sont mémorisés en relation avec les décalages correspondants. Une méthode d'apprentissage est mise en oeuvre qui utilise le jeu de données acquis pour trouver une relation entre les spectres en absorbance et les décalages en longueur d'onde. On utilise par exemple la méthode PLS évoquée plus haut qui conduit à l'établissement d'un modèle de prédiction de décalage en longueur d'onde.

La valeur de décalage estimée par rapprochement avec le modèle de prédiction n'est pas appliquée telle quelle. Elle est combinée, dans un processus itératif, à celle précédemment appliquée, ce qui garantit une certaine stabilité du système, étant supposé que le décalage en longueur d'onde du spectromètre à un instant est fortement corrélé au décalage à un instant immédiatement précédent. On utilise un traitement connu de filtrage par filtre passe-bas itératif (étape 66) qui permet d'élaborer la valeur de décalage à appliquer au spectre brut à partir de la valeur estimée au moyen du modèle de prédiction et de la valeur précédemment appliquée mémorisée en mémoire 68.

Le spectre recalé après l'étape 64 est traité par division, longueur d'onde par longueur d'onde, par le spectre de référence préenregistré en mémoire 70 afin de calculer l'absorbance (étape 72) et fournir un spectre en absorbance.

Un prétraitement (étape 74) pourra éventuellement être effectué sur le spectre en absorbance afin de détecter une mesure aberrante. Une telle mesure aberrante peut provenir d'un mauvais positionnement de la tête de mesure ou d'un positionnement dans une zone particulière non représentative du fruit (par exemple au niveau du pédoncule ou du calice).

La détection d'une mesure aberrante peut être effectuée par un traitement analytique du spectre en absorbance. Un tel traitement consiste par exemple à effectuer une analyse comparative des pentes de la courbe représentative du spectre au voisinage de certaines longueurs d'ondes prédéterminées.

Si une mesure aberrante est détectée, les données spectrales recueillies sont rejetées et une nouvelle mesure doit être effectuée. La détection d'une mesure aberrante peut commander un changement automatique de position de mesure, ou l'affichage d'un message sur le terminal 32 à l'intention d'un opérateur, ou un recyclage du fruit en entrée de l'installation.

Le spectre en absorbance, lorsque la mesure est validée après l'étape 74, est utilisé pour déterminer le décalage en longueur estimé par rapprochement avec le modèle de prédiction correspondant (étape 76).

En outre, ce spectre en absorbance est utilisé pour élaborer une teneur en sucre par rapprochement avec le modèle de prédiction correspondant à la température du fruit analysé. Il peut toutefois être utile de vérifier si la température du fruit analysé n'a pas subi un décalage significatif par rapport à la température d'origine saisie. En effet, une dérive notable en température peut se produire lorsque des mesures sont effectuées sur un lot important de fruits, par exemple lorsque le lot a été extrait d'une chambre froide.

Aussi, la sélection du modèle de prédiction utilisé (étape 78), pour effectuer un rapprochement avec ce modèle (étape 80 de prédiction de teneur en sucre), afin de fournir une valeur de teneur en sucre, est-elle effectuée à partir d'une valeur de température courante prenant en compte une dérive de température éventuelle.

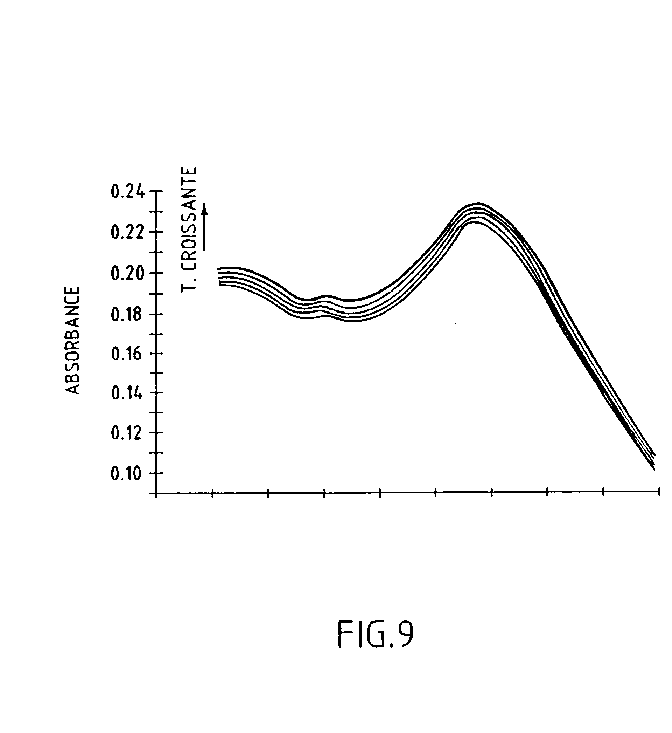

La dérive en température est calculée par rapprochement avec un modèle de prédiction. Ce modèle est élaboré (figure 8) de la même façon que le modèle de prédiction de teneur en sucre ou de décalage en longueur d'onde. Un jeu de données est constitué contenant un lot de spectres acquis sur différents fruits, présentant des taux de sucre différents, à des température différentes entre, par exemple 1,5°C et 35°C. Les spectres recueillis sont traités par le spectre de référence pour fournir un lot de spectres en absorbance qui sont mémorisés en relation avec les valeurs de température correspondantes. La figure 9 montre, pour un fruit, l'évaluation de l'absorbance en fonction de la température. On constate que l'absorbance augmente lorsque la température augmente, et ce de façon significative. Une méthode d'apprentissage, telle que la méthode PLS, est mise en oeuvre pour trouver une relation entre les spectres en absorbance et les températures afin d'élaborer un modèle de prédiction de température.

Le spectre en absorbance obtenu après l'étape 72 est rapproché du modèle de prédiction (étape 82 de prédiction de température) afin de fournir une température estimée. Celle-ci est combinée, au travers d'un filtre passe-bas itératif (étape 84), à la valeur de température précédemment utilisée, afin de fournir une valeur de température courante qui est utilisée pour sélectionner le modèle de prédiction de teneur en sucre et est enregistrée en mémoire 60.

Lorsque deux valeurs au moins de teneur en sucre estimée ont été obtenues lors de deux mesures consécutives, ces deux valeurs sont combinées pour améliorer la précision de la mesure (étape 86). Ainsi, l'écart entre les deux valeurs peut être comparé à un seuil prédéterminé. Si l'écart est inférieur à ce seuil, la moyenne des deux valeurs est adoptée comme valeur de teneur en sucre mesurée. Sinon, une mesure supplémentaire peut être effectuée et la valeur mesurée est la valeur moyenne entre les deux valeurs estimées les plus proches.

Agréage de lot de fruits

On a décrit ci-avant l'évaluation de la teneur en sucre d'un fruit par moyenne de deux mesures effectuées sur le fruit.

Le procédé et l'installation peuvent être aussi utilisés pour l'évaluation de la teneur en sucre d'un lot de fruits par moyenne de plusieurs mesures effectuées sur différents fruits du lot.

Cette évaluation est notamment utile pour le contrôle, ou agréage, d'un lot de fruits reçu ou à expédier.

Dans ce cas, on pourra ne faire qu'une seule mesure par fruit sélectionné dans le lot. En outre, l'estimation de la teneur en sucre est réalisée pour un nombre de fruits du lot généralement supérieur à deux, ce nombre étant choisi en fonction des caractéristiques du lot (nombre de fruits, hétérogénéité apparente, variations de couleurs, exigence de l'agréage, ...). Par conséquent, la valeur finalement obtenue est une valeur moyenne calculée sur généralement plus de deux teneurs estimées.

Autres modes de réalisation

Il a été décrit ci-avant un processus permettant de déterminer une dérive en température, à partir d'une température initialement mémorisée, par rapprochement avec un modèle de prédiction. Il est envisageable de mesurer en permanence la température des fruits afin d'obtenir directement une information représentative de la température courante pour sélectionner le modèle correspondant de prédiction de teneur en sucre.

Dans ce qui précède, on a envisagé le cas où une sélection d'un modèle de prédiction est effectuée en fonction de la température parmi un ensemble de modèles de prédiction préétablis pour différentes températures.

Il est possible, selon une première variante, d'utiliser un modèle de prédiction unique préétabli pour une température donnée, et de corriger la valeur estimée de teneur en sucre obtenue par rapprochement avec ce modèle en fonction d'une loi de variation préétablie pour le type de fruit considéré.

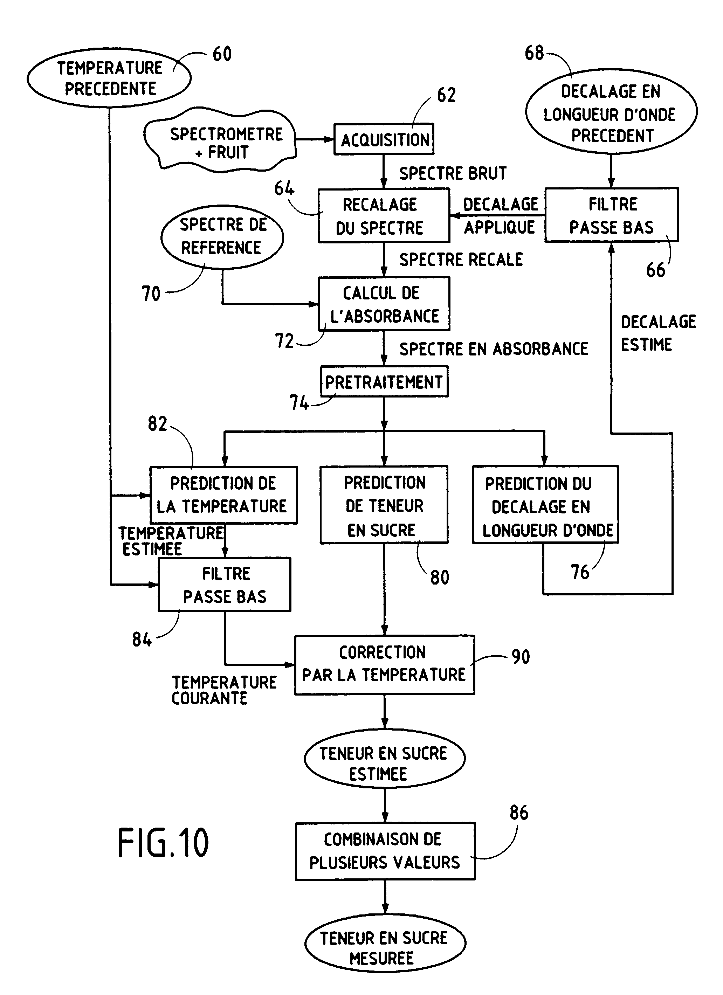

Cette première variante est illustrée par l'ordinogramme de la figure 10 qui se distingue de celui de la figure 6 en ce que le rapprochement avec le modèle de prédiction de teneur en sucre (étape 80) est effectué avec le spectre en absorbance sans sélection préalable d'un modèle en fonction de la température courante, et en ce que le résultat de ce rapprochement est corrigé (étape 90), en fonction de la température courante, selon une loi de correction préétablie, afin de fournir la valeur de teneur en sucre estimée.

La loi de correction est établie de façon expérimentale en relevant, pour un type de fruit donné, la variation de la teneur en sucre estimée par le modèle en fonction de l'écart entre la température courante et une température de référence à laquelle le modèle de prédiction de teneur en sucre a été établi.

Selon une deuxième variante, il est possible d'utiliser plusieurs modèles de prédiction préétablis pour différentes températures pour le type de fruit considéré, et d'effectuer un rapprochement du spectre recueilli avec les différents modèles de prédiction. Le taux de corrélation maximum est obtenu avec le modèle de prédiction correspondant à la température la plus proche de celle des fruits.

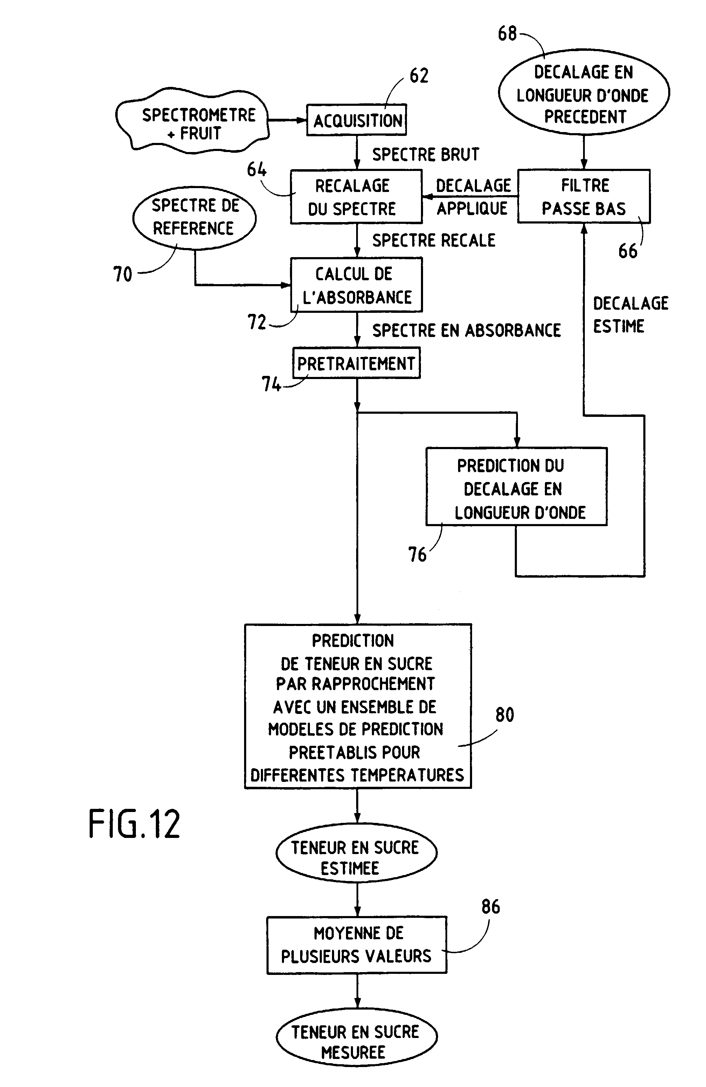

Cette deuxième variante est illustrée par l'ordinogramme de la figure 12 qui se distingue de celui de la figure 6 en ce qu'il n'y a pas de processus d'estimation de la température courante des fruits et de sélection d'un modèle de prédiction en fonction de la température, mais que la prédiction de teneur en sucre (étape 80) est réalisée par rapprochement du spectre recueilli avec des différents modèles de prédiction préétablis.

**Revendications**

1. Procédé de mesure de la teneur de fruits ou légumes en une matière déterminée, comprenant l'illumination de l'intérieur d'un fruit ou légume à travers sa surface par un rayonnement lumineux infrarouge, le recueil de données spectrales représentatives du rayonnement rétrodiffusé par le fruit ou légume et le traitement des données spectrales recueillies pour fournir une valeur de la teneur recherchée par rapprochement avec au moins un modèle de prédiction préétabli, caractérisé en ce qu'une pluralité de mesures sont effectuées par illumination des fruits ou légumes à caractériser au moyen d'un faisceau de fibres optiques dont une extrémité est appliquée sur la surface desdits fruits et légumes, et la valeur de la teneur recherchée est élaborée à partir de la moyenne des mesures effectuées et en s'affranchissant de l'influence de la température réelle desdits fruits ou légumes.

2. Procédé selon la revendication 1, pour la mesure de la teneur en une matière déterminée d'un fruit ou légume pris individuellement, caractérisé en ce qu'au moins deux mesures sont effectuées en deux zones différentes de la surface d'un fruit ou légume.

3. Procédé selon la revendication 1, pour la mesure de la teneur en une matière déterminée d'un lot de fruits ou légumes, caractérisé en ce qu'une pluralité de mesures sont effectuées par illumination de différents fruits ou légumes du lot.

4. Procédé selon l'une quelconque des revendications 1 à 3, caractérisé en ce que plusieurs mesures sont effectuées simultanément.

5. Procédé selon l'une quelconque des revendications 1 à 3, caractérisé en ce que plusieurs mesures sont effectuées séquentiellement.

**6.** Procédé selon l'une quelconque des revendications 1 à 5, caractérisé en ce que le traitement des données spectrales recueillies comprend une sélection d'un modèle de prédiction de teneur en sucre, en fonction de la température, parmi un ensemble de modèles de prédiction préétablis pour différentes températures.

**7.** Procédé selon la revendication 6, caractérisé en ce que la sélection de modèle de prédiction est réalisée en fonction de la température courante mesurée des fruits ou légumes.

**8.** Procédé selon la revendication 6, pour la mesure de la teneur en une matière déterminée d'une succession de fruits ou légumes de même nature, caractérisé en ce que le traitement des données spectrales recueillies comprend une phase d'évaluation, à partir des données spectrales recueillies, d'une dérive éventuelle de la température des fruits ou légumes à partir d'une température initiale mémorisée.

**9.** Procédé selon la revendication 8, caractérisé en ce que l'évaluation de la dérive en température est effectuée à partir d'un rapprochement des données spectrales recueillies avec un modèle de prédiction de température

**10.** Procédé selon l'une quelconque des revendications 1 à 5, caractérisé en ce que le traitement des données spectrales recueillies comprend l'élaboration d'une première valeur de la teneur par rapprochement avec un modèle de prédiction préétabli pour une température donnée, et la détermination de la valeur de la teneur recherchée à partir de la première valeur élaborée, selon une relation prédéterminée fonction de la température du fruit ou légume ou du lot de fruits ou légumes.

**11.** Procédé selon l'une quelconque des revendications 1 à 5, caractérisé en ce que le traitement des données spectrales recueillies comprend le rapprochement des données spectrales avec un ensemble de modèles de prédiction préétablis pour lesdits fruits ou légumes, et pour différentes températures.

**12.** Procédé selon l'une quelconque des revendications 1 à 11, dans lequel l'illumination d'un fruit ou légume et le recueil du rayonnement rétrodiffusé sont réalisés au moyen d'un faisceau de fibres optiques dont une extrémité est amenée au contact de la surface du fruit ou légume, caractérisé en ce que l'extrémité du faisceau de fibres optiques est sollicitée élastiquement vers la surface du fruit ou légume.

**13.** Procédé selon la revendication 12, caractérisé en ce que l'extrémité du faisceau de fibres optiques est orientée lors de son contact avec la surface du fruit ou légume de manière à illuminer la surface dans une direction sensiblement normale à celle-ci.

**14.** Procédé selon l'une quelconque des revendications 12 et 13, caractérisé en ce que le rayonnement rétrodiffusé est reçu dans une sphère d'intégration.

**15.** Procédé selon l'une quelconque des revendications 1 à 14, caractérisé en ce que le rapprochement entre les données spectrales recueillies et un modèle de prédiction est réalisé sur un ensemble limité de longueurs d'onde déterminé par application d'un algorithme génétique.

**16.** Procédé selon l'une quelconque des revendications 1 à 15, caractérisé en ce que le traitement des données spectrales recueillies comprend une phase de détection d'un décalage éventuel en longueur d'onde du spectre du rayonnement rétrodiffusé, par rapprochement entre les données spectrales recueillies et un modèle de prédiction de décalage en longueur d'onde, de manière, en cas de décalage détecté, à effectuer un recalage du spectre avant rapprochement des données spectrales recueillies avec le modèle de prédiction de teneur en sucre.

**17.** Procédé selon l'une quelconque des revendications 1 à 16, caractérisé en ce que le traitement des données spectrales recueillies comprend une phase de détection de mesure aberrante, par analyse d'une ou plusieurs informations tirées des données spectrales recueillies, de manière à rejeter les données spectrales recueillies correspondant à une mesure aberrante.

**18.** Procédé selon l'une quelconque des revendications 1 à 17, caractérisé en ce qu'il comprend une étape d'acquisition d'un spectre de référence par illumination par le rayonnement infrarouge d'une surface réfléchissant sensiblement la totalité du rayonnement, et enregistrement des données spectrales représentatives du rayonnement réfléchi, et une étape de prétraitement de données spectrales recueillies par division par le spectre de référence, afin d'obtenir des données représentatives d'un spectre en absorbance.

**19.** Procédé selon la revendication 18, caractérisé en ce que l'étape d'acquisition du spectre de référence comprend l'illumination d'un objet ayant une forme semblable à celle du fruit ou légume à caractériser.

**20.** Procédé selon l'une quelconque des revendications 1 à 19, caractérisé en ce qu'il comprend une sélection d'un modèle de prédiction correspondant à la nature du fruit ou légume à caractériser parmi un ensemble de modèles de prédiction préétablis pour différentes natures de fruits ou légumes.

21. Installation pour la mesure de la teneur de fruits ou légumes en une matière déterminée, comprenant une source de rayonnement infrarouge (10), un dispositif (20) de spectrométrie infrarouge, des moyens optiques (12, 14, 16 ; 12', 14', 58) reliés à la source et au dispositif de spectrométrie pour illuminer une surface par le rayonnement de la source et acheminer un rayonnement rétrodiffusé au dispositif de spectrométrie, et une unité de traitement (30) reliée au dispositif de spectrométrie pour recueillir un ensemble de données spectrales représentatives d'un rayonnement rétrodiffusé et fournir une valeur de la teneur recherchée par rapprochement avec au moins un modèle de prédiction préétabli, caractérisée en ce que l'unité de traitement (30) comprend des moyens pour fournir la valeur de teneur recherchée par moyenne de mesures à partir d'une pluralité d'ensembles de données spectrales recueillis et en s'affranchissant de l'influence de la température réelle de fruits ou légumes à caractériser, et les moyens optiques comprennent un faisceau de fibres optiques, dont une extrémité libre (16, 12'a) est associée à des moyens de rappel élastique (50) pour solliciter élastiquement l'extrémité du faisceau de fibres vers une surface d'un fruit ou légume.

22. Installation selon la revendication 21, caractérisée en ce que l'extrémité libre (16) du faisceau de fibres optiques est montée librement pivotante sur un support (40) et une pièce de guidage (48) est montée sur la partie de faisceau commun pour permettre, par contact avec une surface, une orientation automatique de l'axe de la partie de faisceau commun dans une direction sensiblement normale à la surface, par pivotement par rapport au support.

23. Installation selon la revendication 21, caractérisée en ce que les moyens optiques comprennent une sphère d'intégration (58), un faisceau de fibres optiques (12') traversant la sphère d'intégration pour amener le rayonnement incident et un faisceau de fibres optiques (14') connecté à la sphère d'intégration pour recueillir le rayonnement rétrodiffusé.

24. Installation selon l'une quelconque des revendications 21 à 23, caractérisée en ce que des moyens sont prévus pour enregistrer un ensemble de modèles de prédiction correspondant à différentes températures d'un fruit ou légume particulier.

25. Installation selon la revendication 24, caractérisée en ce que l'unité de traitement (30) comprend des moyens pour fournir la valeur de teneur recherchée par rapprochement avec un modèle de prédiction sélectionné en fonction de la température des fruits ou légumes.

26. Installation selon la revendication 24, caractérisée en ce que l'unité de traitement (30) comprend des moyens pour fournir la valeur de teneur recherchée par rapprochement avec ledit ensemble de modèles de prédiction.

27. Installation selon l'une quelconque des revendications 21 à 25, caractérisée en ce que l'unité de traitement (30) comprend des moyens pour fournir une valeur de teneur par rapprochement avec un modèle de prédiction préétabli pour une température donnée et correction de la valeur fournie selon une relation prédéterminée fonction de la température des fruits ou légumes.

28. Installation selon l'une quelconque des revendications 25 et 27, caractérisée en ce que l'unité de traitement (30) comprend des moyens permettant une correction automatique d'une information de température pré-enregistrée par détection d'une dérive de température à partir d'informations tirées des données spectrales recueillies.

29. Installation selon l'une quelconque des revendications 21 à 28, caractérisée en ce que l'unité de traitement (30) comprend des moyens permettant une détection de décalage en longueur d'onde d'un spectre de rayonnement reçu par le dispositif de spectrométrie (20).

30. Installation selon l'une quelconque des revendications 21 à 29, caractérisée en ce que l'unité de traitement comprend des moyens permettant de détecter une mesure aberrante à partir d'informations tirées des données spectrales recueillies.

31. Installation selon l'une quelconque des revendications 21 à 30, caractérisée en ce que des moyens sont prévus pour enregistrer des modèles de prédiction correspondant à différentes natures de fruits ou légumes.

FIG.1

FIG.2

FIG.3

ILLUMINATION D'UN
OBJET DE REFERENCE

↓

PRISE DE PLUSIEURS
MESURES

↓

ELABORATION DU
SPECTRE DE REFERENCE

## FIG.4

ACQUISITION DE SPECTRES
SUR DES FRUITS

↓

ELABORATION DE SPECTRES
EN ABSORBANCE

↓

MESURE DESTRUCTIVE
DES TENEURS EN SUCRE

↓

APPRENTISSAGE

↓

ETABLISSEMENT DE
MODELE DE PREDICTION
DE TENEUR EN SUCRE

## FIG.5

DECALAGE DU SPECTROMETRE
EN LONGUEUR D'ONDE

↓

ACQUISITION DE SPECTRES
SUR DES FRUITS

↓

ELABORATION DE SPECTRES
EN ABSORBANCE

↓

APPRENTISSAGE

↓

ETABLISSEMENT DE
MODELE DE PREDICTION
DE DECALAGE EN
LONGUEUR D'ONDE

## FIG.7

ACQUISITION DE SPECTRES
A DIFFERENTES TEMPERATURES

↓

ELABORATION DE SPECTRES
EN ABSORBANCE

↓

APPRENTISSAGE

↓

ETABLISSEMENT DE
MODELE DE PREDICTION
DE TEMPERATURE

## FIG.8

TEMPERATURE PRECEDENTE 60

SPECTROMETRE + FRUIT → ACQUISITION 62

SPECTRE BRUT

DECALAGE EN LONGUEUR D'ONDE PRECEDENT 68

RECALAGE DU SPECTRE 64 ← DECALAGE APPLIQUE ← FILTRE PASSE BAS 66

SPECTRE DE REFERENCE 70

SPECTRE RECALE

CALCUL DE L'ABSORBANCE 72

DECALAGE ESTIME

SPECTRE EN ABSORBANCE

PRETRAITEMENT 74

PREDICTION DE LA TEMPERATURE 82

PREDICTION DU DECALAGE EN LONGUEUR D'ONDE 76

TEMPERATURE ESTIMEE

FILTRE PASSE BAS 84

TEMPERATURE COURANTE

SELECTION DU MODELE CORRESPONDANT A LA TEMPERATURE 78

FIG.6

PREDICTION DE TENEUR EN SUCRE 80

TENEUR EN SUCRE ESTIMEE

MOYENNE DE PLUSIEURS VALEURS 86

TENEUR EN SUCRE MESUREE

14

FIG.9

TEMPERATURE PRECEDENTE — 60

DECALAGE EN LONGUEUR D'ONDE PRECEDENT — 68

SPECTROMETRE + FRUIT → ACQUISITION — 62

SPECTRE BRUT

RECALAGE DU SPECTRE — 64

DECALAGE APPLIQUE ← FILTRE PASSE BAS — 66

SPECTRE DE REFERENCE — 70

SPECTRE RECALE

CALCUL DE L'ABSORBANCE — 72

DECALAGE ESTIME

SPECTRE EN ABSORBANCE

PRETRAITEMENT — 74

PREDICTION DE LA TEMPERATURE — 82

PREDICTION DE TENEUR EN SUCRE — 80

PREDICTION DU DECALAGE EN LONGUEUR D'ONDE — 76

TEMPERATURE ESTIMEE

FILTRE PASSE BAS — 84

CORRECTION PAR LA TEMPERATURE — 90

TEMPERATURE COURANTE

TENEUR EN SUCRE ESTIMEE

FIG.10

COMBINAISON DE PLUSIEURS VALEURS — 86

TENEUR EN SUCRE MESUREE

FIG.11

SPECTROMETRE + FRUIT → ACQUISITION 62

SPECTRE BRUT

68 DECALAGE EN LONGUEUR D'ONDE PRECEDENT

64 RECALAGE DU SPECTRE ← DECALAGE APPLIQUE ← FILTRE PASSE BAS 66

SPECTRE DE REFERENCE 70

SPECTRE RECALE

72 CALCUL DE L'ABSORBANCE

SPECTRE EN ABSORBANCE

DECALAGE ESTIME

74 PRETRAITEMENT

PREDICTION DU DECALAGE EN LONGUEUR D'ONDE 76

PREDICTION DE TENEUR EN SUCRE PAR RAPPROCHEMENT AVEC UN ENSEMBLE DE MODELES DE PREDICTION PREETABLIS POUR DIFFERENTES TEMPERATURES 80

FIG.12

TENEUR EN SUCRE ESTIMEE

86 MOYENNE DE PLUSIEURS VALEURS

TENEUR EN SUCRE MESUREE